# EUROPÄISCHE PATENTANMELDUNG

(11) **EP 0 774 273 A2**
(43) Veröffentlichungstag der Anmeldung: **21.05.1997**
(21) Anmeldenummer: 96118234.2
(22) Anmeldetag: 14.11.1996
(51) Int. Cl.: A61N 1/36

(54) **Gerät sowie Verfahren zur Diagnostik, Leistungssteigerung und zur Wiederherstellung gestörter Nerven- und Muskelaktivitäten**

(30) Priorität: 20.11.1995 DE 19543211
(71) Anmelder: Haynl, Kurt, 39221 Eggersdorf (CH)
(72) Erfinder: Haynl, Kurt, 39221 Eggersdorf (DE); Paerisch, Manfred, Prof. Dr. med. habil., 04277 Leipzig (DE); Richter, Jens, 39218 Schönebeck (DE)
(74) Vertreter: Leinung, Günter

(57) **Zusammenfassung**

Das Gerät zur Erzeugung eines Elektrostimulationssignales ist so aufgebaut, daß die Impulsstromeinleitung und die elektromyographische Ableitung körpereigener Signale über die gleiche Elektrodenapplikation erfolgt und mit einem Microcomputer verbunden ist, mit dem ein variables Zeitregime, wie unterschiedliche Stromimpulsdauer, Stromimpulspausen, Impulsgruppendauer, Impulsgruppenperioden und Trainingseinheiten bestimmt werden können, wobei wesentlich ist, daß die Stromimpulse in verschiedener Polarität erzeugt und abgegeben werden.

## Beschreibung

Die Erfindung bezieht sich auf ein Gerät sowie ein physiologisches Verfahren zur Diagnostik, Leistungssteigerung sowie zur Wiederherstellung gestörter Nerven- und Muskelaktivitäten des menschlichen Körpers mittels impulsförmiger Ströme.

Das Gerät ist unmittelbar mit einem Programmiersystem koppelbar und so ausgebildet, daß unter Anwendung von elektrischen Strömen und elektrischen Feldänderungen auf biologische Gewebsstrukturen, wie Muskel- und Nerven, zu diagnostischen und therapeutischen Zwecken eingewirkt wird.

Die Anwendung von elektrischen Strömungen und Spannungen auf biologische Gewebsstrukturen, wie Muskeln und Nerven, zu diagnostischen und therapeutischen Zwecken ist allgemein bekannt.
So werden in dem Hilfsmittelverzeichnis der Krankenkassen unter der Produktgruppe 09 Elektrostimulationsgeräte aufgeführt, deren Definitionsbereiche für anzuwendende Mittelfrequenzströme von 1 kHz bis 10 kHz angegeben sind. Die meisten zur Zeit verwendeten Reizstromgeräte erzeugen periodische Impulsströme innerhalb dieses Frequenzbereiches.

So bezieht sich auch die Veröffentlichung EU-0203336 A1 auf ein Reizstromgerät sowie Verfahren zur Behandlung des menschlichen und tierischen Körpers mittels impulsförmiger Ströme, die über Elektroden appliziert werden, welches so ausgebildet ist, daß es während mindestens einer Impulsgruppendauer jeweils nur Impulse einer einzigen, wählbaren Polarität abgibt.

Den bekannten Lösungen haftet der Nachteil an, daß sowohl die Verfahren als auch die bekannten Vorrichtungen mit gleichen, sich wiederholenden Impulsen innerhalb einer Impulsgruppe arbeiten.

Aufgabe der vorliegenden Erfindung ist es, ein Gerät sowie ein Verfahren zur Diagnostik, Leistungssteigerung und zur Wiederherstellung gestörter Nerven- und Muskelaktivitäten zu entwickeln, die die Nachteile der Behandlung mit gleichen Impulsen innerhalb einer Impulsgruppe nicht aufweisen und in zu wählenden Grenzen innerhalb einer kleinsten vorgebbaren Zeiteinheit die Erzeugung von Impulsstromgruppen ermöglichen.

Die Aufgabe wird erfindungsgemäß durch die Merkmale der Ansprüche 1 und 5 gelöst.

Besondere Ausgestaltungen und vorteilhafte Lösungen sind Gegenstand von Unteransprüchen.

Das Gerät zur Erzeugung eines Elektrostimulationssignales ist so aufgebaut, daß die Impulsstromeinleitung und die elektromyographische Ableitung körpereigener Signale über die gleiche Elektrodenapplikation erfolgt und mit einem Microcomputer verbunden ist, mit dem ein variables Zeitregime, wie unterschiedliche Stromimpulsdauer, Stromimpulspausen, Impulsgruppendauer, Impulsgruppenfolgeperioden und Trainingseinheitenzeiten bestimmt werden können.

Das mit diesem Gerät realisierbare psychologische Verfahren zur Behandlung gestörter Nerven- und Muskelaktivitäten beruht auf dem Verständnis der Wirkungen von eingeleiteten Stromimpulsen auf Körpergewebe und beachtet dabei, daß diese Körpergewebe sowohl eine Teilchenstruktur, nämlich Elektronen, Atome, Moleküle und damit verbunden auch eine elektrische Struktur, nämlich die Verteilung elektrischer Ladungen an Grenzflächen, besitzen.

Das Hauptmerkmal dieser Strukturen ist der Nicht-Gleichgewichtszustand (NGZ) bezüglich der Verteilung elektrischer Ladungen an den Grenzflächen zellulärer und subzellulärer Strukturelemente, welcher nur unter Energieaufwand aufrecht erhalten werden kann.

Außerdem ist dieser Zustand durch die Überlagerung elektrischer Felder der Ladungsträger gekennzeichnet.

Die Zufuhr elektrischer Ladungen durch Elektrostimulation kann zum einen den bestehenden NGZ verstärken (Hyperporalisation), den Übergang zu einem neuen NGZ fördern oder auf die Einstellung des Gleichgewichtszustandes hinwirken, was nicht erreicht werden soll.

Das bedeutet, elektrische Impulse größerer Breite und längerer zeitlicher Dauer fördern über eine Grenzflächen-Depolarisation die Einstellung des Gleichgewichtszustandes und setzen so die Funktionsfähigkeit der Gewebe herab oder schädigen diese irreversibel. Kann die ursprüngliche Funktion des Gewebes nicht wieder hergestellt werden, ist dies nur mit einem sehr hohen Energieaufwand möglich, so daß gemäß des vorgeschlagenen erfinderischen Verfahrens zur Elektrostimulation elektrische Impulse sehr kurzer zeitlicher Dauer auf das zu behandelnde Gewebe einwirken. Diese Impulse können die energiegestützte Trägheit des NGZ nicht überwinden und führen nur zur Verstärkung der temperaturbedingten, lokalen Fluktuationen der Ladungen. Erreichen die Fluktuationsamplituden einen kritischen Schwellwert, geht das Grenzflächensystem in einen neuen NGZ über, welcher weiter vom Gleichgewichtszustand entfernt ist als der vorherige NGZ. Das System paßt sich an und kann neue Strukturen ausbilden, die leistungsfähiger sind als die vorangegangenen.

Dies bedeutet, daß neue Energieformen in der jeweiligen Gewebezelle entstehen, die höher liegen als die alte Energieform, was wiederum bedeutet, daß dieses System weiter vom Gleichgewichtszustand entfernt ist.

Mit dieser vorgeschlagenen Verfahrensweise kann eine effektive Anwendung von elektrischen Impulsen mit extrem kurzer Impulsdauer und genau einstellbaren Impulsabständen sowie einer einstellbaren Impulspolarität erfolgen. Das bedeutet die Dämpfung kritischer Fluktuationsamplituden und die Beschleunigung von Repolarisationsvergängen.

Zu beachten ist dabei auch, daß der Informationsinhalt zur Erzielung einer Muskelantwort in biologischen Systemen nicht in der zeitlichen Dauer des Impulsstromes, sondern in den Pausen zwischen kurzen Stromimpulsen enthalten ist und dadurch eine Informationsübertragung mit minimalstem Energieaufwand erfolgt.

Durch die Erzeugung von Impulsstromgruppen mit beliebigen Impulsen und Impulspausen innerhalb einer Impulsgruppe bei einer kleinsten vorgebbaren Zeiteinheit wird ermöglicht, daß der Gewöhnungs- oder auch Lerneffekt der Nerven und der Muskulatur auf gleichförmige Reizstromimpulse und somit einer verminderten Reizwirkung des Elektrostimulationsstromes, entgegengewirkt wird.

Die Erfindung wird mit nachfolgendem Ausführungsbeispiel näher erläutert.

Die dazugehörige Zeichnung zeigt in:
- Fig. 1:: ein Impulsdiagramm der Elektrostimulationsparameter mit dem Impulsstromverlauf
- Fig. 2:: ein Impulsdiagramm gesetzter Impulse in unterschiedlicher Zeitfolge
- Fig. 3:: Funktionsschaubild bei der "EMG"
- Fig. 4:: Funktionsschaubild bei der Kombination von der elektromyographischen Erfassung "EMG" Elektrostimulation "ES"
- Fig. 5:: Funktionsschaubild bei der Elektrostimulation "ES"
- Fig. 6:: die Schaltungsanordnung des Gerätes mit Ankopplung an ein Programmiersystem.

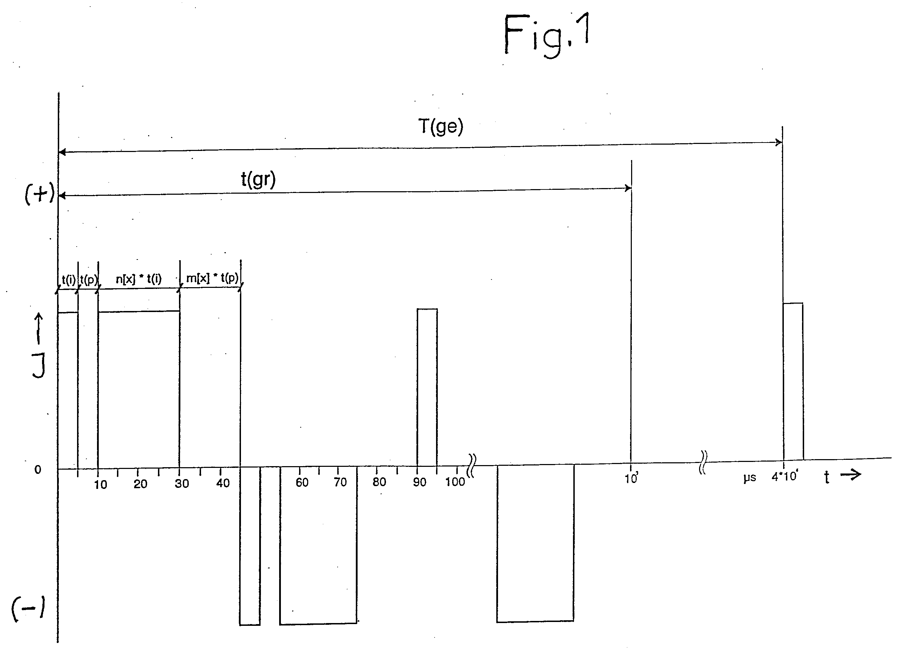

Das Impulsdiagramm nach Figur 1 verdeutlicht, daß mit der vorgestellten erfinderischen Lösung Stromimpulse mit unterschiedlichen Impulsparametern erzeugt werden können, welche im Rahmen eines physiologischen Verfahrens zur Behandlung gestörter Nerven- und Muskelaktivitäten genutzt wird.

Es wird gezeigt, daß innerhalb einer Impulsgruppendauer t (gr) Stromimpulse t (i), in unterschiedlicher Breite und Polarität, erzeugt werden und es wird gleichzeitig verdeutlicht, daß die Stromimpulspausen t (p), als Maß der aufeinanderfolgenden Stromimpulse t (i), sich aus der mit "n" angegebenen Variablen, multipliziert mit dem jeweiligen Stromimpuls t (i), ergibt.
Es wird also deutlich, daß mit dieser Verfahrensweise Stromimpulse t (i) in vorbestimmter Zeitdauer, Intensität und verschiedener Polarität innerhalb einer Impulsgruppendauer erzeugt werden können und innerhalb der Dauer einer Impulsgruppenfolgeperiode t (ge) wiederholbar nachvollzogen werden können.

Unmittelbar zum Verfahren gehört weiterhin, daß die Dauer der Übungsphase T (Ü) und die Dauer der Teilübung T (T) eingestellt werden können, wobei die Parameter auf verschiedenen Übungsprogrammen über ein entsprechendes Programmiersystem eingegeben werden. Mit dieser Verfahrensweise kann somit eine effektive Anwendung von kurzen elektrischen Stromimpulsen t (i) sowie den genauen einstellbaren Stromimpulsen t (p) und einer einstellbaren Impulspolarität erfolgen, wodurch eine Dämpfung kritischer Fluktuationsamplituden und die Beschleunigung von Repolarisierungsvorgängen erreicht wird.

Praktisch bedeutet dies, daß bei kurzer Stromimpulsefolge der jeweils angesteuerte bzw. gereizte Nerv oder Muskel die Information differenziert, was zur Folge hat, daß die Nerven- und Muskelfunktionen aktiviert werden und somit beispielsweise eine Steigerung der Kontraktionsschnelligkeit bzw. zu beschleunigten Muskelkontrkationen führt.

Eine Impulsfolge mit zeitlich größeren Impulspausen führt zu einer gleichförmigen Muskelkontraktion, d.h. zur Summation der Information.

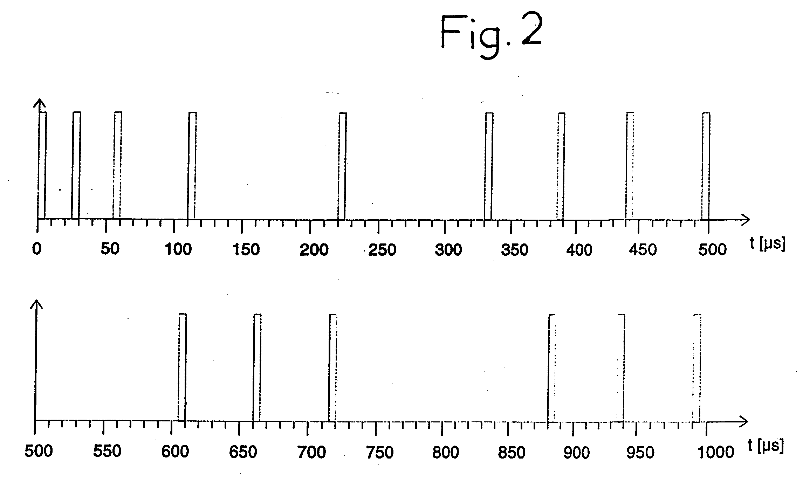

Das in der Figur 2 dargestellte Impulsdiagramm verdeutlicht nochmals über einen Zeitstrahl die bildliche Wiedergabe der gesetzten Stromimpulse t (i) in einem Zeitabstand kleiner 10 µs und bei unterschiedlichen Stromimpulspausen t (p).

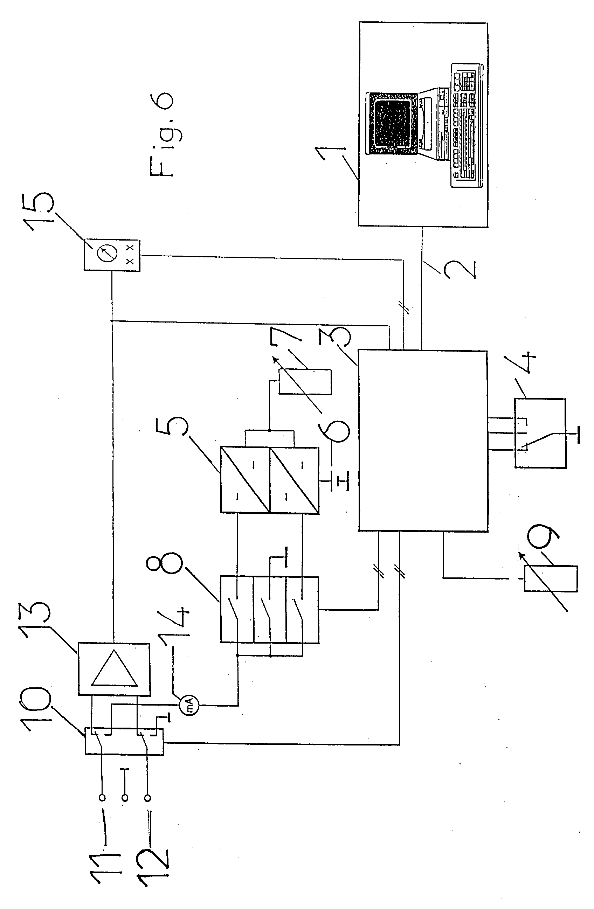

Das Gerät zur Erzeugung der Stromimpulse wird in seinem Aufbau und seiner schaltungstechnischen Verknüpfung aus der Figur 6 ersichtlich, welches über eine Schnittstelle 2 mit einem Programmiersystem 1 gekoppelt werden kann.

Das Kernstück des Gerätes ist ein Mikrocomputer 3, welcher so ausgelegt ist, daß verschiedene Stimulations- und Übungsprogramme gespeichert und nach Wahl entsprechend eines angeschlossenen Programmschalters 4 abgearbeitet werden können.
Der vorgesehene Einstellregler 9 ist für die weitere Anwendung dieses Gerätes im Rahmen der Elektrostimulation vorgesehen, deren Anwendung weiter unten näher beschrieben wird.

Der Microcomputer 3 ist schaltungstechnisch mit einem Anzeigegerät 15 und mit einem Umschalter 10 verbunden, welcher mit entsprechenden Ausgängen 11, 12 ausgeführt ist. Innerhalb des Gerätes befindet sich eine Stromversorgung 6, ein Sollwertgeber 7, welcher mit der Spannungsquelle 5 verbunden ist und diese Spannungsquelle 5 steht wiederum in Verbindung mit einem Schalter 8, der ausgangsseitig über einen Strommesser 14 mit dem Umschalter 10 verbunden ist. Über einen Verstärker 13 ist dieser Umschalter 10 gleichfalls mit dem Anzeigegerät 15 verbunden, so daß unmittelbar vom Anzeigegerät 15 die erreichbaren elektromyographischen Muskel-Aktionspotentiale, die mit den an den Ausgängen 11, 12 angeordneten Elektroden vom jeweils zu reizenden Gewebe angezeigt werden, gleichzeitig ist die Zeitdauer der Teilübungen ablesbar.

Zum funktionellen Ablauf:

Mit dem Programmiersystem 1, bestehend aus einem PC und spezieller Software, werden alle erforderlichen Daten für die Erzeugung der Impulsparameter, wie die Breite und Polarität der einzelnen Stromimpulse t (i), die Breite der einzelnen Stromimpulspausen t (p), die Dauer der Elektrostimulationsphase, der Impulsgruppendauer t (gr) als auch die Dauer der Impulsgruppenfolgeperiode t (ge) und die Dauer der Übungs- und Teilübungsphasen T (Ü) und T (T) eingestellt und über die Schnittstelle 2 auf den Mikrocomputer 3 übertragen.

Dabei können verschiedene Parameter und verschiedene Übungen mit diesem gesamten Gerät übertragen werden, wobei die gesamte Übungszeit sich aus der Dauer und der Zeit der einzelnen Teilübungen der Elektrostimulation (ES) und der elektromyographischen Stimulation (EMG) ergibt und in der Regel zwischen 10 und 30 Minuten andauert.

Der Mikrocomputer 3 ist in seiner Arbeitsweise so ausgelegt, daß verschiedene Stimulations- und Übungsprogramme gespeichert und nach Wahl entsprechend des Programmschalters 4 abgearbeitet werden.

Ein bipolare Spannungsquelle 5, hier als bipolarer Schaltregler ausgeführt, wandelt die Batteriebetriebsspannung der Stromversorgung 6 in die erforderlichen Ausgangsspannungen (bis + 400 V und - 400 V) und -ströme (bis + 500 mA und -500 mA), wobei Konstantspannungs- oder Konstantstrombetrieb möglich ist. Die Ausgangsparameter Spannung oder Strom werden innerhalb der Regelungsgrenzen durch den Sollwertgeber 7 gewählt.

Mit den Schaltern 8, gesteuert vom Microcomputer 3 entsprechend des gespeicherten Programms der Impulsparameter, werden die Elektrostromimpulse erzeugt, mit dem Strommesser 14 gemessen und über den Umschalter 10 und den Ausgängen 11 und 12 der Elektrodenapplikation dem menschlichen Körper zugeführt. Der Umschalter 10 wird ebenfalls vom Mikrocomputer 3 entsprechend der programmierten Zeiten für den Übungsablauf gesteuert und schaltet nach der Übungsphse der Elektrostimulation die am Ausgang 11 und 12 angeschlossenen Elektroden über den Umschalter 10 an den Verstärker 13 und bringt die willentliche elektromyographische Muskelspannung auf dem Anzeigegerät 15 zur Anzeige.

Der Verstärker 13 ist dabei als sogenannter EMG-Verstärker, bestehend aus den Baugruppen Präzisionsmeßverstärker, Filtereinheiten und Meßgleichrichter, ausgeführt. Mit der Anzeigeeinheit 15 werden die erreichbaren elektromyographischen Muskel-Aktionspotentiale, die mit den Elektroden der Ausgänge 11 und 12 vom menschlichen Körper abgeleitet werden, und die Zeitdauer der Teilübungen anzeigt.

Wahlweise besteht programmintern mit dem Mikrocomputer 3 die Möglichkeit, durch ein vom Ausgang des Verstärkers 13 auf dem Mikrocomputer 3 geführten Signales, welches dem willentlich erzeugten elektromyographischen Muskelpotentials der EMG-Abteilung proportional ist, dazu zu verwenden, über den Mikrocomputer 3 den Umschalter 10 derart zu triggern, daß nach einer willentlichen Muskelkontraktion und einer wählbaren (Trigger) Schwelle mit dem Einstellregler 9 die Elektrostimulation der gleichen Muskelgruppe ausgelöst wird, wenn das initierte EMG-Signal einen mit dem Einstellregler 9 vorbestimmten Wert überschreitet.

Mit dem neuen Stimulationsgerät ist es somit möglich, die Wirkung verschiedener programmierbarer Stromimpulse auf das Körpergewebe zu untersuchen, wobei die besondere Gerätekonfiguration dem Bedienenden und Benutzer optisch und akustisch die Zustände in den einzelnen Stimulationsphasen verdeutlicht.

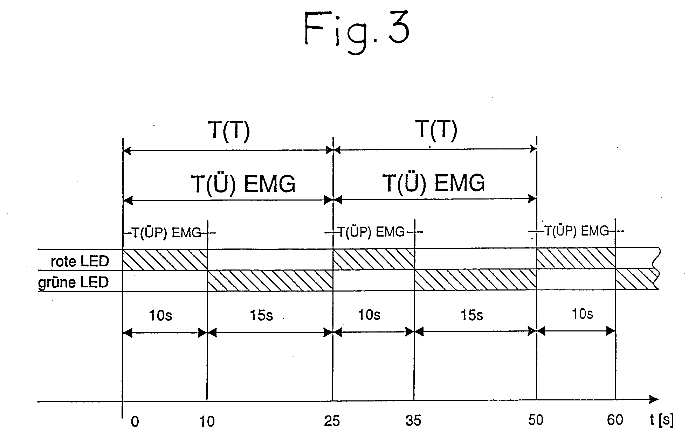

Die Figur 3 gibt dabei Auskunft über den Funktionsablauf bei der Elektromyographischen Stimulation (EMG) innerhalb einer Übungsphase.
Um den Betrachter bzw. Bedienenden auch optisch den jeweiligen Zustand zu verdeutlichen, wird unmittelbar die Reizung durch eine rote Leuchtdiode (LED) angezeigt, während die grüne Leuchtdiode die Pause zwischen zwei Reizungen angibt.

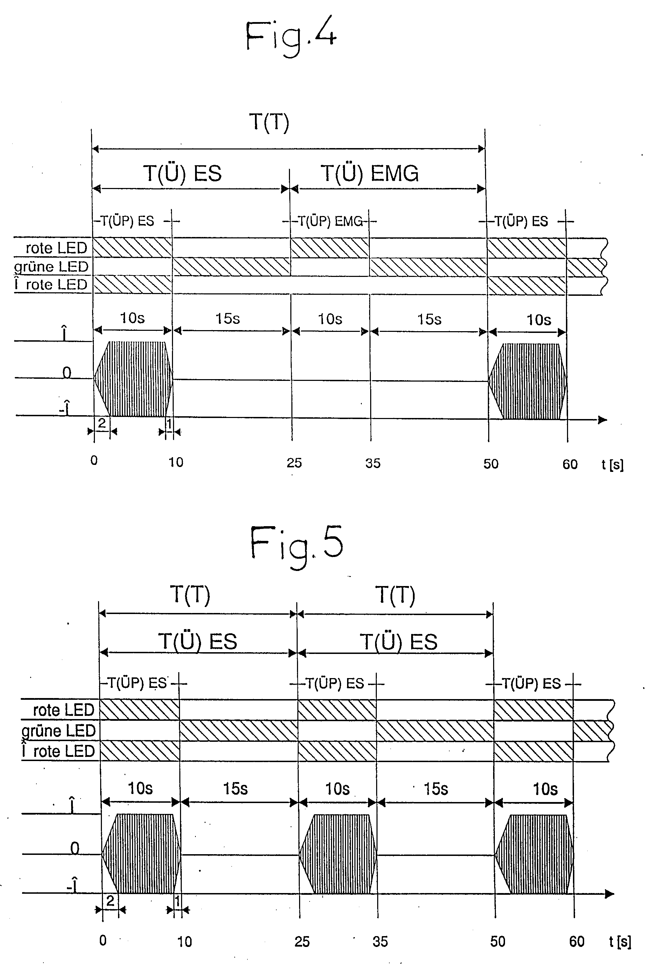

In analoger Weise wird in den Figuren 4 und 5 die optische Wiedergabe des jeweiligen Reizzustandes und die Methode gezeigt, wobei die Figur 4 unmittelbar auf ein Übungsprogramm bezug nimmt, bei der die Übung in kombinierter Weise aus der Elektrostimulation (ES) und der elektromyographischen Stimulation (EMG) besteht. Auch hier zeigen die vorgesehenen roten und grünen Leuchtdioden den jeweiligen Zustand des Reizens oder der Pausen an.

Die Figur 5 verdeutlicht dabei den Ablauf innerhalb der Elektrostimulation (ES), welche gleichfalls über die vorgesehenen Leuchtdioden den jeweiligen Zustand signalisieren.

Das erfindungsgemäße Verfahren und Stimulationsgerät werden eingesetzt, um gestörte Nerven- und Muskelaktivitäten zu diagnostizieren, wieder herzustellen und um Funktionssteigerungen dieser Gewebe anzustreben. Das Anwendungsgebiet ist dabei sehr weit gefächert.
In der Geronthologie erfolgt der Einsatz zur Verhinderung der Beschränkung altersbedingter Funktionseinbußen von Nerven und Muskeln, beispielsweise Skelettmuskelatropie, Inkontinenzschwächen von Darm und Blase. Im Sport kann der Einsatz zur Wiederherstellung leistungsangepaßter Muskelfunktionen nach Trauma und Operation sowie zur Steigerung der Kontraktionsschnelligkeit und der Energieübertragung in Muskelaggregaten erfolgen, wobei weitere Anwendungsfälle durchaus denkbar sind.

## Patentansprüche

1. Gerät zur Erzeugung von elektrischen Stromimpulsen für geschwächte Nerven- und Muskelgewebe, die über Elektroden appliziert werden und welches in unterschiedlichen Betriebsarten arbeitet, dadurch gekennzeichnet, daß
- programmierbare Einzelstromimpulse t (i) und programmierbare Stromimpulspausen t (p) innerhalb einer Stromimpulsgruppendauer t (gr) erzeugt und abgegeben werden,
- die Stromimpulse t (i) eine gleiche oder unterschiedliche Polarität aufweisen,
- eine Impulsgruppendauer t (gr) aus beliebigen programmierbaren Stromeinzelimpulsen n (x) * t (i) und Stromimpulspausen m (x) * t (p) besteht, wobei
- die Variablen (n und m) den Stromimpulsen t (i) und den Stromimpulspausen t (p) zugeordnet sind und
- deren Impulsdauer kleiner 10 µs ist.

2. Gerät nach Anspruch 1, dadurch gekennzeichnet, daß
das Gerät über eine Schnittstelle (2) mit einem Programmiersystem (1) verknüpft ist und innerhalb des Gerätes ein Mikrocomputer (3) vorgesehen ist, welcher einen Programmschalter (4) und einen Einstellregler (9) besitzt und mit einem Umschalter (10) und einem Anzeigegerät (15) verbunden ist.

3. Gerät nach den Ansprüchen 1 und 2, dadurch gekennzeichnet, daß
innerhalb des Gerätes eine Spannungsquelle (5), eine Stromversorgung (6), ein Sollwertgeber (7) sowie ein Schalter (8) angeordnet sind, wobei der Schalter (8) mit dem Mikrocomputer (3) und über einen Strommesser (14) mit dem Umschalter (10), welcher Ausgänge (11,12) besitzt, verbunden ist und zwischen dem Umschalter (10) und dem Anzeigegerät (15) ein Verstärker (13) angerodnet ist.

4. Verwendung des Gerätes nach den Ansprüchen 1 bis 3 zur Erzeugung von elektrischen Stromimpulsen für folgende Zwecke:
zur Diagnostik, Leistungssteigerung und zur Wiederherstellung gestörter Nerven- und Muskelaktivitäten, sportliches Muskeltraining, Wiederherstellung und Funktionssteigerungen von gestörten Nerven- und Muskelaktivitäten, Beseitigung der Inkontinenzschwäche von Darm und Blase.

5. Verfahren zur Diagnostik, Leistungssteigerung und zur Wiederherstellung gestörter Nerven- und Muskelaktivitäten und zur physiologischen Überprüfung der Muskelgewebe, dadurch gekennzeichnet, daß
- die Stromimpulseinleitung und die elektromyographische Ableitung körpereigener Signale über die gleiche Elektrodenapplikation erfolgt,
- das Verfahren nach einem variablen Zeitregime abläuft und bestimmt wird nach unterschiedlicher Stromimpulsdauer t (i), den Stromimpulspausen t (p), der Impulsgruppendauer t (gr), der Stromimpulsgruppenfolgeperiode t (ge) sowie der Dauer der Übungsphasen T (Ü) und T (T), wobei
- die Stromimpulse so auf das Körpergewebe wirken, daß der Nichtgleichgewichtszustand (NGZ) dieser Strukturen bezüglich der Verteilung elektrischer Ladungen an den Grenzflächen zellulären und subzellulären Strukturelemente gefördert wird und
- die elektrischen Stromimpulse aufbestimmbare Impulsmengen, Impulsintervallen von unterschiedlicher Impulspolarität eingestellt werden können.

6. Gerät nach Anspruch 5, dadurch gekennzeichnet, daß
die Elektrostimulation (ES) in Kombination mit der Messung elektromyographischer Signale (EMG) zur physiologischen Überprüfung von Muskelgewebe verwendet wird.

7. Verfahren nach den Ansprüchen 5 und 6, dadurch gekennzeichnet, daß
Stromimpulse t (i) innerhalb einer Impulsgruppendauer t (gr) in unterschiedlichen vorprogrammierbaren Stromimpulspausen t (p) und in unterschiedlicher Polarität gesetzt werden können.

8. Verfahren nach den Ansprüchen 5 bis 7, dadurch gekennzeichnet, daß
die Stromimpulsdauer unter 10 µs liegt.
